# EUROPEAN PATENT SPECIFICATION

(11) **EP 4 181 844 B1**
(45) Date of publication and mention of the grant of the patent: **25.11.2026**
(21) Application number: 21843498.3
(22) Date of filing: 14.07.2021
(51) Int. Cl.: A61F 9/007, A61F 9/00

(54) **OCULAR ACOUSTIC DEVICE**
AKUSTISCHE AUGENVORRICHTUNG
DISPOSITIF ACOUSTIQUE OCULAIRE

(30) Priority: 14.07.2020 US 202063051657 P
(43) Date of publication of application: 24.05.2023
(73) Proprietor: Tel Hashomer Medical Research, Infrastructure and Services Ltd., 5265601 Ramat-Gan (IL); Bar-Ilan University, 5290002 Ramat Gan (IL)
(72) Inventor: SKAAT, Alon, 7531387 Rishon LeZion (IL); LESHNO, Ari, 6291797 Tel Aviv (IL); KENIGSBERG, Avraham, 9987500 Tzur Hadassah (IL); SHPAISMAN, Hagay, 4485600 Kedumim (IL)
(74) Representative: Modiano, Gabriella Diana
(86) International application number: PCT/IL2021/050862
(87) International publication number: WO 2022/013871

(56) References cited:
- US-A1- 2010 152 626
- US-A1- 2017 027 751
- US-A1- 2017 087 014
- US-A1- 2017 087 014

## Description

### TECHNOLOGICAL FIELD

The present invention is directed to the field of devices for ocular treatment and diagnosis.

### BACKGROUND

Ophthalmic disorders of various degrees of severity and potential for short and/or long-lasting damage to functioning of the eye often involve abnormal presence of particulate matter in the aqueous and/or vitreous fluid of the eye. For example, whereas floaters that typically accumulate in the vitreous fluid of the eye may be annoying and interfere with clarity of vision, they are relatively benign. On the other hand, particulate matter, such as pigment cells exfoliated from the iris and blood particles caused by injury and/or disease may generate lasting and irreversible damage to the eye. Such particulate matter in the eye's anterior chamber for example may drift to the eye's trabecular meshwork and/or Schlemm's canal, interfere with circulation of aqueous fluid in the eye, and cause glaucoma. Abnormal presence of particular matter is exhibited by such pathological conditions as exfoliation syndrome (XFS), pigmentary dispersion, Uveitis-Glaucoma-Hyphemia (UGH), and asteroid hyalosis.

Various ophthalmic conditions might result in formation or dispersion of particles of different shapes, sizes and trait circulation inside the aqueous humor. In addition to obscuration of the visual axis in the acute phase, these particles might also interact with intraocular tissues resulting in long-term adverse effects such as secondary glaucoma and corneal staining that might lead to irreversible loss of vision. Currently there are no means to change the clinical course with no way to control or even moderate the extent and duration of particle dispersion (regardless of etiology) and the main treatment course in such cases involves only close observation for early detection and treatment of complications.

US2017087014 discloses methods and apparatus, including eyewear and a contact lens, for delivering sound energy to an eye, comprising two or more sonic or ultrasonic transducers that emit sound energy, wherein each transducer is (i) operably linked to a power source and (b) capable of emitting sound energy at more than one frequency and for a variable time period; and a positioning mechanism to position the transducers at an exterior surface of an eye so as to deliver sound energy to an internal part of the eye, for example the Schlemm's canal or trabecular meshwork.

US2010152626 discloses a n apparatus for delivering sound energy to an eye, comprising: (a) two or more transducers that emit sound energy and are operably linked to a power source, wherein each transducer is independently capable of emitting sound energy at more than one frequency and for a variable time period; and (b) a positioning mechanism to position the transducers at an exterior surface of an eye so as to deliver the sound energy to an internal part of the eye.

US2017027751 discloses a device for treatment of an ocular pathology characterized in that it comprises at least one eye ring wherein the proximal end of said eye ring is suitable to be applied onto the globe and means to generate ultrasound beam fixed on the distal end of the eye, said means to generate ultrasound beam presenting a concave segment shape conformed along a single curvature corresponding to a single direction wherein the concavity is designed to be tuned towards the eyeglobe.

### GENERAL DESCRIPTION

Intra-ocular particles carry both short and long-term morbidity. There is a need in the art to provide a technique for moderating or preventing damage resulting from accumulation and dispersion of particulate matter in the eye. Various conditions result in accumulation of pathologic particles within the anterior chamber. The interaction between the particles and the intraocular tissues (particularly the angle), lead to secondary open-angle glaucoma with very high intraocular pressure and a more serious clinical course and worse prognosis. These particles cause both visual disturbances and limits examination ability. Therefore, there is also a need in the art to provide a technique for displacing particulate matter in the eye to be able, clearing the particles away from the visual axis and reducing their accumulation within the trabecular meshwork by concentrating them in a designated area, thus preventing secondary open angle glaucoma. It should be noted that the particles obscure vision and instead of waiting for them to sink with gravity (which can in some cases take days), the particles are manually displaced by using the teachings of the present invention downwards and clear the visual axis to reduce the disturbance.

A diagnosis may be performed by examining the movement (e.g. speed or trajectory) of the particulate matter under an acoustic field by using the teachings of the present invention enabling distinguishing between various types of particles such inflammatory, pigmentary and red blood cells.

The present invention enables assisting in manipulation and movement of intraocular particles in order to treat various ocular conditions. This may be implemented by manipulating the particles for clearing the visual axis as described above and/or preventing secondary open angle glaucoma. As noted above, over time the particles accumulate and interact with the trabecular meshwork tissue resulting in structural and functional damage causing reduced outflow and increased intraocular pressure. The superior portion of the angle may be protected by moving the particles downwards. It should be noted that although more particles aggregate in the inferior half of the angle, keeping at least a third of the angle open is sufficient to prevent a rise in intraocular pressure.

It enables controlling intra-ocular particles using acoustic wave forces. The novel technique of the present invention utilizes acoustic radiation forces for non-invasive control over movement of the pathologic intraocular particles, preventing them from unwanted accumulation and obstruction of intraocular structures, or causing visual disturbances. Acoustic forces can be harnessed to non-invasively organize various types of endogenous and exogenous particles within the anterior chamber of the eye, without any sign of tissue damage. Acoustic forces can easily and relatively quickly arrange particles within the anterior chamber. The particles aggregation is capable of remaining stable, despite deactivation of the acoustic wave. The novel technique of the present invention is operable for applying acoustic trapping and manipulation methods based on acoustic radiation force that enables control over movement of intraocular particles. In this way, visual disturbance caused pathologic particles may be solved. This technique may be used as a preventive treatment for accumulation and obstruction of intraocular structures. By manipulating the particle movement, it is possible to hasten recovery and clearing of the visual axis with quicker return to normal vision. In this way, unwanted accumulation of these particles is prevented in the trabecular meshwork and reduce the risk for secondary Glaucoma. This technique helps relief of symptoms and also minimizes, if not prevent, secondary complications. In addition, the use of acoustic manipulation enables to provide a non-invasive, fast (i.e. in the order of seconds) and non-harmful technique in which the particles/cells are influenced simultaneously. The relevant acoustic frequencies and amplitudes result in acoustic fields that are considered safe to live organic tissues. The technique of the present invention can be used in various ocular conditions involving intraocular particles such as exfoliation syndrome (XFS), pigmentary dispersion, hyphema, vitreous floaters, asteroid hyalosis as well as other ocular conditions in which pathologic particles are present in the anterior or posterior chambers.

According to one broad aspect of the present invention, there is provided an ocular acoustic device used for manipulation of intraocular particles within the anterior and posterior chambers of the eye by applying directional acoustic energy in specific patterns. The intraocular particles can be either moved to or from a desired location. Moreover, the external shape formed by the intraocular particles may be configured based on predetermined acoustic parameters. The predetermined acoustic parameters are selected to enable movement of the particles within the eye without damaging the eye's internal tissues. The predetermined acoustic parameters comprise (but are not limited to) at least one of acoustic frequencies, excitation amplitudes and phases being considered safe to live organic tissues. The predetermined acoustic parameters may also comprise the configuration of the acoustic radiation pattern defining parallel or focused beam of acoustic energy into the eye based on the acoustic contrast factor. The acoustic contrast factor generally describes whether a given type of particle in a given medium is attracted to the pressure nodes or antinodes of a wave pattern. The sign of the acoustic contrast factor is defined by the relationship between densities and sound velocities (or, equivalently densities and compressibilities) of particles and the eye media. Particles that are more dense and less compressible than the surrounding medium (thus having a positive acoustic contrast factor) are driven to the nodal areas due to scattering of the standing acoustic wave. The size of specific particles can influence magnitude of the applied acoustic force. The device is thus configured to direct particles within the anterior chamber to predetermined locations resulting in hastened recovery and relief of symptoms and minimization if not prevention of secondary complications.

An aspect of an embodiment of the disclosure relates to providing an ocular acoustic device and method for generating and using a directional acoustic energy creating an acoustic pressure to manage particulate matter present in the anterior posterior, and/or vitreous chamber of the eye that may affect health of the eye.

An aspect of an embodiment of the disclosure relates to providing a device for generating and configuring acoustic radiation, which may be advantageous for controlling movement and concentration of particles present in the aqueous fluid and/or vitreous humor of the eye.

There is therefore provided in accordance with an embodiment of the disclosure device for controlling movement and position of particles present in an eye, the acoustic device comprising: an acoustic transducer being configured and operable to generate acoustic waves upon excitation and a controller being configured and operable to excite vibrations in the acoustic transducer to generate acoustic waves that enter and produce an acoustic field in the eye having a directional acoustic energy in a certain pattern that operates to apply forces to the particles.

In an embodiment the acoustic device operates to generate and apply forces to the particles that operate to cause the particles to drift to and/or cluster at particular regions of the eye, or to attract or repel one another.

In an embodiment the acoustic transducer comprises a single or a plurality of acoustic transducers. The acoustic transducer may thus be configured as a probe-like device allowing control over the direction of particle movement by displacement (e.g. changing the probes' location along a three-dimensional path). Alternatively, this may be implemented by using a combination of multiple acoustic resonators. A synchronized activation procedure may then allow controlling over particle movement. The acoustic transducer may also be configured a phased array of acoustic transducers configured to be placed on the cornea of the eye and couple acoustic energy into the eye.

According to the claimed invention, the controller is configured and operable for generating and configuring acoustic radiation for a certain period of time and capable of controlling movement and concentration of particles by controlling at least one acoustic parameter and the certain period of time. The controlling of the movement and concentration of particles comprises causing the particles to drift to and/or cluster at particular regions of the eye, or to attract or repel one another. Optionally the controller is operable to control at least one acoustic parameter comprising excitation amplitudes and/or phases or frequencies of each transducer so that the acoustic transducers operate as a phased array to generate the acoustic field in the eye. The controller may thus control the phased array to generate desired configurations of acoustic radiation.

In an embodiment the acoustic device comprises a collimating lens that receives the acoustic waves generated by the acoustic transducer to generate a collimated beam of traveling wave acoustic energy.

In an embodiment the acoustic device comprises a motion stage being capable of holding the acoustic transducer and being configured and operable to controllably position and manipulate the acoustic transducer on the eye. The position and the motion of the acoustic field can be controlled along at least five degrees of freedom.

In an embodiment the acoustic device is configured and operable to generate acoustic standing waves defining nodes and antinodes regions. The controller is operable to excite vibrations in the acoustic transducer at a frequency that generates an acoustic standing wave pattern in the eye. The acoustic standing wave pattern may comprise at least one nodal region to which the particles are attracted and trapped. The controller may be operable to modify the position of the nodes in the acoustic standing wave pattern It should be noted that acoustic manipulation enables particle movement by acoustic radiation forces. When a fluid with foreign solid particles is irradiated by a standing sound field, the suspended particles experience steady (time-averaged) hydrodynamic forces which make them drift or cluster at certain space points.

In this connection it should be noted that the invention is not limited to any specific shape of the nodes pattern. Radial or circular nodes as well as straight lines may be used. For example, the acoustic standing wave is characterized by at least one substantially circular nodal region in which the particles in the eye are trapped. Optionally, the acoustic standing waves exhibit substantially circularly concentric nodal and anti-nodal regions in the aqueous fluid of the anterior eye chamber and/or posterior eye chamber, and/or in the vitreous humor of the vitreous chamber. Optionally, the acoustic device is configured to generate an acoustic radiation exhibiting a Cₙ rotationally symmetric nodal pattern of degree "n" rotational symmetry.

Alternatively, the acoustic device is configured to generate a traveling wave of acoustic radiation. The acoustic field may comprise an acoustic traveling wave having a relatively high acoustic intensity waist region. Optionally the at least one region to which the particles are attracted and trapped comprises the waist region. In an embodiment the controller controls the amplitudes and/or phases of the transducers to move the at least one region and particles trapped in the at least one region to a desired region in the eye.

In an embodiment the acoustic device is configured to generate a collimated beam of acoustic energy. To this end, the device may comprise an acoustic lens that receives the acoustic waves generated by the acoustic transducer and collimates the waves into a plane wave.

In an embodiment the acoustic device is configured to generate a beam of traveling wave acoustic energy focused to a desired internal location in the eye.

In an embodiment the acoustic device is configured to generate a focused beam of traveling acoustic waves that lead to movement of particles by acoustic streaming. The acoustic streaming is capable of moving or dislodging the particles from specific locations or streamed towards desired locations in the eye.

In an embodiment, the acoustic device is configured to generate an acoustic wave that excites the cornea to create vibrations that in turn induce a standing corneal acoustic wave within the anterior chamber of the eye. In this connection, it should be understood that the acoustic wave is configured in such a way that, when impinging on the external surface of the eye, the acoustic wave is reflected by the cornea and boundary conditions that meet the criteria for formation of a secondary standing waves are met. The appropriate selection of the angle at which the acoustic wave is projected onto the eye and of the three-dimensional position of the acoustic transducer relatively to the eye (e.g. distance between the acoustic transducer and the eye) enables to fulfill the conditions for reflection of the acoustic wave and creation of the corneal standing acoustic wave due to the circular configuration of the eye. This could be achieved at any frequency.

In some embodiments, the ocular acoustic device further comprises a coupling layer being configured and operable to physically couple between the acoustic transducer and the eye and provide acoustic impedance match thereof. The coupling layer may be configured and operable to fit the surface of eye so that each of acoustic transducers is acoustically coupled to the eye. The coupling layer may be configured and operable to seal the acoustic transducer to the eye so that the transducer is capable of being filled with a liquid.

In some embodiments, the controller is configured and operable to excite vibrations in the acoustic transducer to generate a nonlinear focused traveling acoustic wave with a finite amplitude creating an acoustic streaming dislocating the particles.

In an embodiment the device comprises an acoustic hologram that receives acoustic waves generated by the acoustic transducer and configures the received wave to form the acoustic field in the eye. The configured acoustic wave generated by the acoustic hologram is encoded such that the particles may be displaced in the eye. In this way, the movement of the particles may be controlled without physically displacing the transducer. The acoustic hologram may be configured by the controller.

In some embodiments, the acoustic hologram may be configured to receive traveling acoustic plane waves generated by a substantially planar acoustic transducer such that the desired acoustic field is created at a desired distance from the hologram.

In an embodiment the device comprises a housing that houses at least one or any combination of more than one of the acoustic transducers, acoustic lens, and/or hologram. Optionally, the motion stage is configured to hold the housing and to control its position on the eye to facilitate entry of the acoustic waves into the eye and production of the acoustic field. Additionally or alternatively the housing may be configured to be manually manipulated on the eye.

Optionally, the housing is stylus shaped and the coupling layer comprises a contact tip configured to be placed on at least one portion of the eye and to couple acoustic energy from the acoustic transducer into the eye.

In an embodiment the acoustic device comprises a stylus-like implement having a contact tip configured to be placed on the cornea of the eye and couple acoustic energy from the generator into the eye. The stylus may be operable to radiate a parallel or focused beam of acoustic energy into the eye.

Optionally the acoustic transducer has an external shape defining a semi closed-loop (i.e. a closed-loop configuration has an aperture) such as a cylindrical shell. The aperture of the acoustic transducer enables to displace the acoustic transducer around the eye. Optionally the cylindrical shell transducer is formed as a single monolithic unit.

In an embodiment, changing the position of the acoustic transducer enables to affect different regions of the eye. Therefore, the acoustic field may be located in at least one of an anterior chamber of the eye or a posterior chamber of the eye. In an embodiment, the acoustic field comprises an acoustic field located in the vitreous chamber of the eye.

In some embodiments, the controller is configured and operable to determine a starting position of the acoustic transducer, a scanning path, a scanning speed and at least one acoustic parameter of the acoustic field according to an image data.

In some embodiments, the ocular acoustic device further comprises an imaging module being configured and operable to collect image data being indicative of the displacement of the particles. The controller may be connected to the imaging module for receiving the image data.

According to another broad aspect of the present invention, there is provided a method for controlling movement and position of particles present in an eye. The method comprises generating acoustic waves being capable to enter into the eye; producing an acoustic field in the eye having a directional acoustic energy in a certain pattern that operates to apply forces to the particles; and controlling movement of particles to at least one predetermined location.

In some embodiments, generating acoustic waves comprises at least one of generating an acoustic standing wave in the eye; generating corneal standing acoustic waves; generating acoustic traveling waves or generating a plurality of acoustic waves having different acoustic parameters.

In some embodiments, generating acoustic a plurality of acoustic waves having different acoustic parameters comprises generating a plurality of excitation voltages to increase a thickness of agglomerated particles.

In some embodiments, producing an acoustic field in the eye comprises at least one of controlling at least one acoustic parameter of the acoustic waves to configure the acoustic radiation or generating a collimated beam of acoustic energy.

In some embodiments, controlling movement of particles to at least one predetermined location comprises manipulating an acoustic device on a surface of an eye to control movement and position of particles present in the eye.

In some embodiments, controlling movement and position of particles present in the eye comprises at least one of displacing particles and controlling concentration of particles at at least one particular region of the eye. This may enables diagnosing various ocular conditions.

In some embodiments, producing an acoustic field in the eye enables treating various ocular conditions.

### BRIEF DESCRIPTION OF THE DRAWINGS

In order to better understand the subject matter that is disclosed herein and to exemplify how it may be carried out in practice, embodiments will now be described, by way of non-limiting example only, with reference to the accompanying drawings, in which:
**Figs. 1A-1B** schematically show perspective and perspective cutaway views respectively of the eye exhibiting features of the eye;
**Fig. 1C** schematically shows an enlarged cross-section of the eye and flow patterns of aqueous fluid from the ciliary body to the trabecular mesh and Schlemm's canal;
**Fig. 1D** schematically shows a cross-section of the eye exhibiting presence of particles in the anterior chamber of the eye that drift towards the trabecular mesh and Schlemm's canal and interfere with circulation of the aqueous fluid;
**Fig. 1E** is a block diagram schematically illustrating the main functional elements of the ocular acoustic device of the present invention;
**Fig. 2A** schematically shows a perspective view of the eye, the abnormal presence of the particles shown in **Fig. 1D****,** and an acoustic device in accordance with an embodiment of the disclosure;
**Fig. 2B** schematically shows the acoustic device shown in **Fig. 2A** agglomerating particles shown in the aqueous fluid of **Fig. 2A** at antinodes of a standing wave pattern and distancing the particles from the trabecular mesh and Schlemm's canal, in accordance with an embodiment of the disclosure;
**Fig. 2C** schematically shows the standing wave pattern generated by excitation of an acoustic transducer and the agglomeration of the particles at nodes of the standing wave caused by the standing wave pattern, in accordance with an embodiment of the disclosure;
**Fig. 2D** shows a schematic perspective view of the eye showing the particles agglomerated at concentric nodes of the standing wave pattern, in accordance with an embodiment of the disclosure;
**Fig. 2E** is a graph that show increase as a function of time of thickness of a ring of agglomerated particles trapped in a region of a circular node of a standing acoustic wave similar to that shown in **Fig. 2D** generated in an enucleated porcine eye by using the teachings of the present invention an acoustic device, in accordance with an embodiment of the disclosure;
**Fig. 2F** shows a picture of an acoustic device comprising a motion stage in accordance with an embodiment of the disclosure;
**Fig. 3** schematically shows an acoustic device comprising a plurality of transducers, operable to generate different configurations in accordance with an embodiment of the disclosure;
**Fig. 4** schematically shows a stylus shaped acoustic device trapping a particle, in accordance with an embodiment of the disclosure; and
**Fig. 5** is a flow chart schematically illustrating the main functional steps of the method for controlling movement and position of particles present in an eye in accordance with an embodiment of the disclosure.

### DETAILED DESCRIPTION OF EMBODIMENTS

In the discussion, unless otherwise stated, adjectives such as "substantially" and "about" modifying a condition or relationship characteristic of a feature or features of an embodiment of the disclosure, are understood to mean that the condition or characteristic is defined to within tolerances that are acceptable for operation of the embodiment for an application for which it is intended. Wherever a general term in the disclosure is illustrated by reference to an example instance or a list of example instances, the instance or instances referred to, are by way of non-limiting example instances of the general term, and the general term is not intended to be limited to the specific example instance or instances referred to. The phrase "in an embodiment", whether or not associated with a permissive, such as "may", "optionally", or "by way of example", is used to introduce for consideration an example, but not necessarily a required configuration of possible embodiments of the disclosure. Unless otherwise indicated, the word "or" in the description and claims is considered to be the inclusive "or" rather than the exclusive or, and indicates at least one of, or any combination of more than one of items it conjoins.

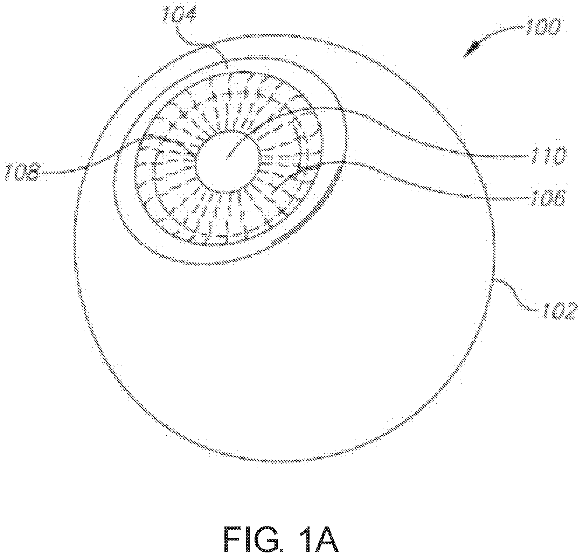

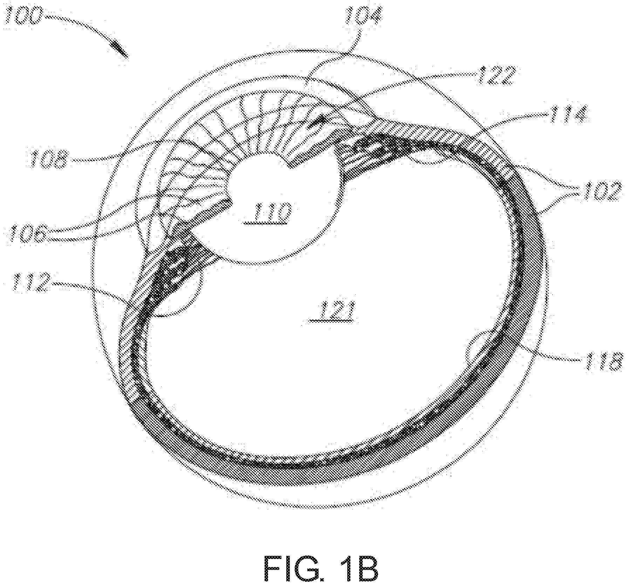

**Fig. 1A** schematically shows a perspective view of a complete human eyeball **100** showing outer features of the eye: the sclera **102;** cornea **104;** iris **106;** perimeter **108** of the pupil (also referred to by numeral **108);** and lens **110** of the eye. **Fig. 1B** schematically shows a perspective, partial cutaway view of eye **100** that shows, in addition to the outer features of the eye shown in **Fig. 1A****,** also internal features of the eye. Internal features of the eye schematically shown in **Fig. 1B** include: the zonular fibers **112** that attach to the capsule (not shown) of lens **110;** the ciliary body **114** in which aqueous fluid is produced; and the retina **118.** A region **121** of eye **100** on a side of lens **110** opposite iris **106** is filled with the vitreous humor (not shown) and is referred to as the vitreous chamber of the eye. A region **122** of eye **100** between iris **106** and cornea **104** is filled with the aqueous fluid (not shown) and is referred to as the anterior chamber of the eye.

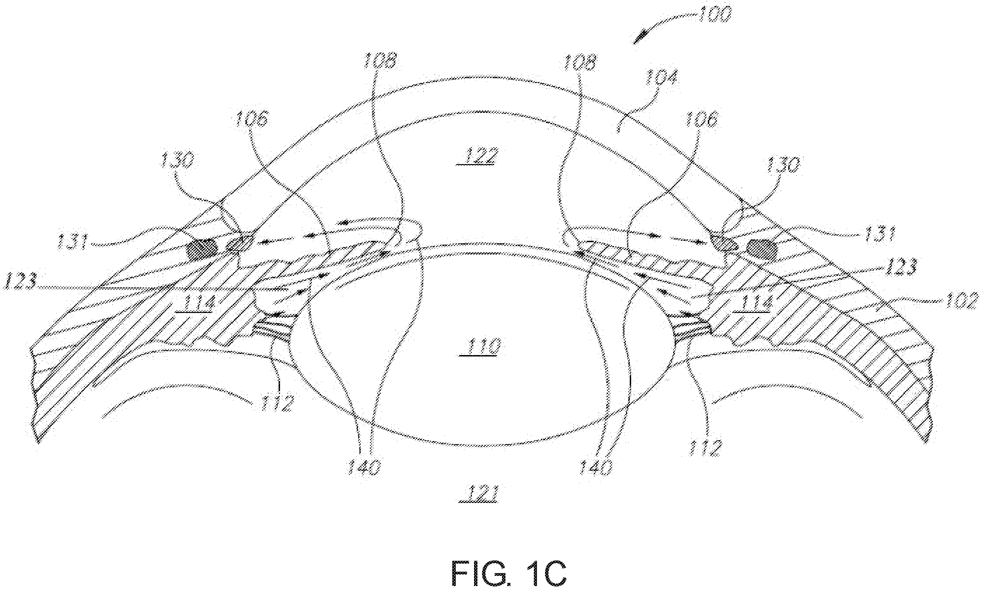

**Fig. 1C** schematically shows an enlarged cross section of a portion of eye **100** that includes anterior chamber **122,** lens **110,** zonular fibers **112** and ciliary body **114** shown in **Figs. 1A** and **1B****.** A region **123** of eye **100** between iris **106** and lens **110** is filled with the aqueous fluid (not shown) and is referred to as the anterior chamber of the eye. In addition, **Fig. 1C** schematically shows trabecular mesh **130** and Schlemm's canal **131.** Trabecular mesh **130** drains aqueous fluid, which is produced in ciliary body **114** and flows into anterior chamber **122,** from the anterior chamber, and delivers the drained aqueous fluid to Schlemm's canal. Aqueous fluid drains from Schlemm's canal into blood vessels (not shown) of the blood stream via aqueous veins (not shown). The aqueous drainage system of the trabecular mesh **130** and Schlemm's canal **131** operates to control flow of aqueous fluid produced in ciliary body **114** and maintain safe internal hydrostatic pressure of the eye. A pattern of flow of aqueous fluid from ciliary body **114,** around iris **106** through pupil **108** to trabecular mesh **130** is schematically represented by arrows **140.** Blockage of trabecular mesh **130** and/or Schlemm's canal **131** disrupts normal flow of aqueous fluid in the eye and may lead to increase of internal hydrostatic pressure in the eye and resultant glaucoma that can lead to blindness.

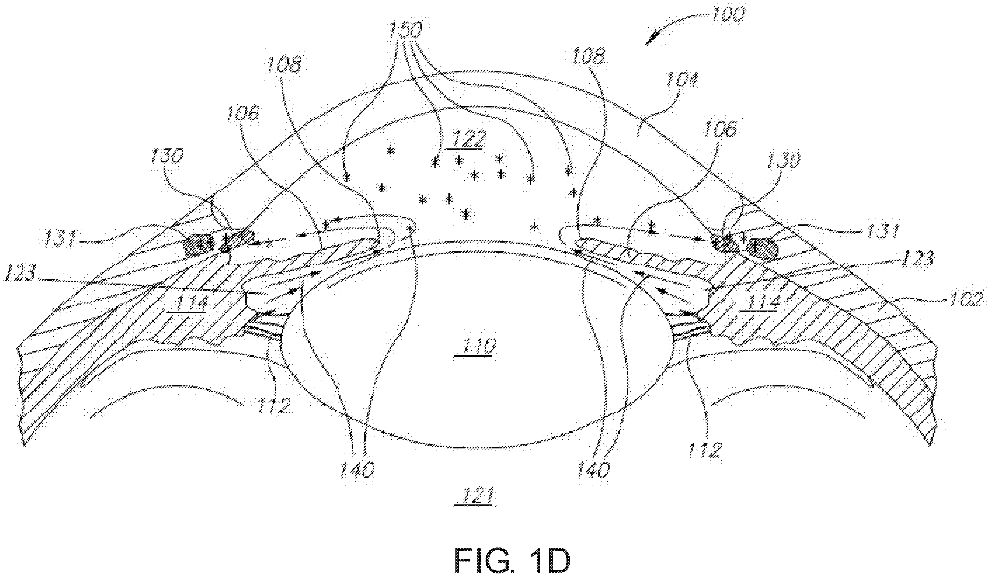

**Fig. 1D** schematically shows the cross section of eye **100** shown in **Fig.** 1C with anomalous presence of particles, represented by asterisks **150,** in anterior chamber **122** that may be carried by flow **140** of aqueous fluid to block trabecular mesh **130** and/or Schlemm's canal **131,** disrupt proper flow of the aqueous fluid, and lead to glaucoma.

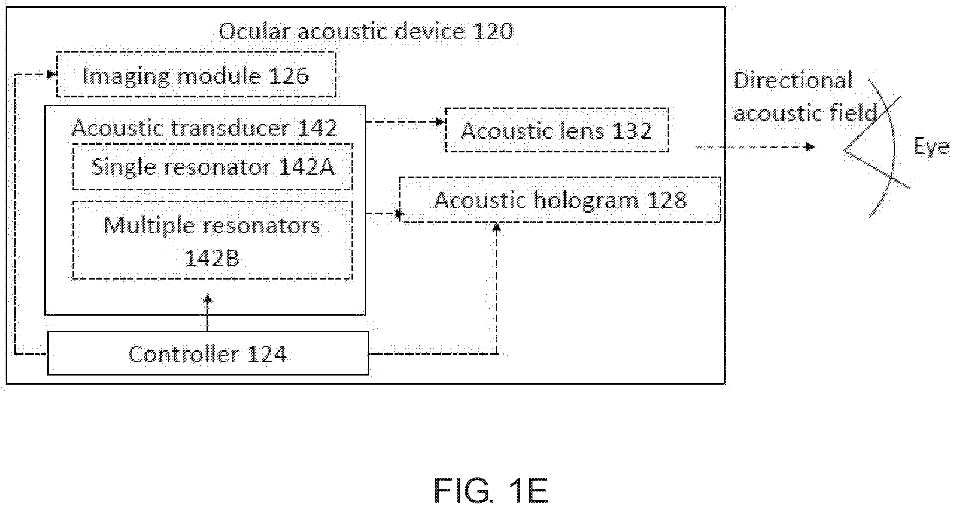

**Fig. 1E** is a block diagram schematically showing an ocular acoustic device **120** for use in controlling movement and position of particles. Ocular acoustic device **120** comprises an acoustic transducer **142** being configured and operable to generate acoustic waves upon excitation and a controller **124** being connected to acoustic transducer **142** and being configured and operable to excite vibrations in the acoustic transducer **22** generating acoustic waves being capable to enter into the eye **100** and produce an acoustic field in the eye **100** having a directional acoustic energy in a certain pattern that operates to apply forces to the particles.

In an embodiment ocular acoustic device **120** may be moved to move the acoustic pattern that the ocular acoustic device **120** produces. Acoustic transducer **142** may be configured as a probe-like device being configured and operable to control over the direction of particle movement by displacement. Acoustic transducer **142** may be for example an electromagnetic, electroacoustic or ultrasound acoustic transducer **142** formed from a ceramic element such as a piezoelectric material.

Acoustic transducer **142** may comprise at least one (e.g. a single) acoustic resonator **142A** or a plurality of acoustic transducers and reflectors **142B** (e.g. synchronized resonators). Each of the acoustic transducer may be independently excitable by controller **124** to vibrate at selectable acoustic parameters including amplitudes, frequencies, and phases of vibrations. Controller **124** may be operated to control at least one acoustic parameter of a single or a plurality of transducers **224** to transmit acoustic waves that generate acoustic wave patterns in eye and manipulate the pattern. The pattern may comprise standing or travelling waves pattern and is not limited to any geometrical shape. It may have a continuous external shape or comprises a plurality of pulse waves. If a standing wave pattern is generated, controller **124** may manipulated the pattern to move location of nodes in the standing wave pattern. For example, in an embodiment, controller **124** controls acoustic transducer(s) **142** to generate an acoustic standing wave pattern configuration having radial dependence of a Bessel function of the first kind that traps and agglomerates particles in nodal regions of the Bessel function. Controller **124** optionally dynamically changes phases and/or amplitudes of the vibrations of transducer(s) **142** to move the Bessel function pattern and its nodes with the trapped and agglomerated particles change to desired locations in the eye for which their potential damage to the eye is moderated.

In some embodiments, ocular acoustic device **120** may comprise a substantially planar acoustic transducer that generates a traveling acoustic plane wave and an acoustic phase plate conventionally referred to as an acoustic holographic plate, or acoustic hologram **128,** on which the acoustic plane wave is incident. The acoustic hologram **128** may be designed to generate a desired acoustic field at a desired distance from the hologram in response to the incident planar wave. In an embodiment the acoustic hologram **128** is configured to generate an acoustic wave front that converges toward a desired high acoustic intensity focal waist that may be used as an acoustic tweezer to trap and control movement of particles in the eye. Acoustic hologram **128** may be implemented by using a 3D-printed surface profile encoding the phase of the desired wavefront. Acoustic hologram **128** may be monolithic and mat reconstruct diffraction-limited acoustic pressure fields and thus arbitrary ultrasound beams.

Additionally or alternatively, ocular acoustic device **120** may comprise an acoustic lens **132** being placed to the output of the acoustic transducer **142** and being configured and operable to receive the acoustic waves generated by the acoustic transducer and generate a collimated beam of the received wave into a plane wave for traveling or standing wave acoustic energy. In this way, a beam of traveling or standing wave acoustic energy can be focused to a desired internal location in the eye.

Controller **124** may comprise a power supply being in the form of a function generator being configured to generate different types of electrical waveforms over a wide range of frequencies. The mode of operation of controller **124** is not limited to any configuration and may be operated manually, automatically, continuously or intermittently according to the conditions required by the manipulation of each type of particles and of the path that the particles should be moved on. Controller **124** is configured and operable for generating and configuring acoustic radiation being capable of controlling movement and concentration of particles. The controlling of the movement and concentration of particles includes causing the particles to drift to and/or cluster at particular regions of the eye, or to attract or repel one another. The controllable acoustic parameters of controller **124** are typically the amplitude (e.g. AC excitation voltages), phase, or frequencies of the acoustic transducer. For example, the particles may be concentrated into one desired region by rotating the acoustic transducer around the eye.

Controller **124** may comprise a general-purpose computer processor, which is programmed in software to carry out the functions described hereinbelow. Also, operations in accordance with the teachings herein may be performed by a computer specially constructed for the desired purposes or by a general-purpose computer specially configured for the desired purpose by a computer program stored in a computer readable storage medium. The software may be downloaded to the controller in electronic form, over a network, for example, or it may alternatively be provided on tangible media, such as optical, magnetic, or electronic memory media. Alternatively or additionally, some or all of the functions of the controller may be implemented in dedicated hardware, such as a custom or semi- custom integrated circuit, or a programmable digital signal processor (DSP). The term controller refers to a computer system, state machine, processor, or the like, designed to perform arithmetic or logic operations using logic circuitry that responds to and processes the instructions that drive a computer.

The technique of the devices according to the present invention can find applicability in a variety of computing or processing environments, such as computer or process-based environments. The techniques may be implemented in a combination of software and hardware. The techniques may be implemented in programs executing on programmable machines such as stationary computers being configured to obtain raw log data, as has also been described above. Program code is applied to the data entered using the input device to perform the techniques described and to generate the output information. The output information can then be applied to one or more output devices.

Each program may be implemented in a high-level procedural or object-oriented programming language to communicate with a processed based system. However, the programs can be implemented in assembly or machine language, if desired.

In other embodiments, the technique of the present invention can be utilized over a network computing system and/or environment. Several computer systems may be coupled together via a network, such as a local area network (LAN), a wide area network (WAN) or the Internet. Each method or technique of the present invention as a whole or a functional step thereof could be thus implemented by a remote network computer or a combination of several. Thus, any functional part of ocular acoustic device can be provided or connected via a computer network. In addition, the controller can also remotely provide processor services over a network.

Each such program may be stored on a storage medium or device, e.g., compact disc read only memory (CD-ROM), hard disk, magnetic diskette, or similar medium or device, that is readable by a general or special purpose programmable machine for configuring and operating the machine when the storage medium or device is read by the computer to perform the procedures described in this document. The ocular acoustic device may also be implemented as a machine-readable storage medium, configured with a program, where the storage medium so configured causes a machine to operate in a specific and predefined manner.

In an embodiment, controller **124** applies voltage at certain frequencies to transducer **142** to excite transverse vibration modes in transducer **142** that generate standing waves.

Additionally or alternatively, controller **124** is configured and operable to excite vibrations in the acoustic transducer generating acoustic waves exciting the cornea to create vibrations that in turn induces a corneal standing acoustic wave within the anterior chamber of the eye.

Additionally or alternatively, controller **124** may excite transducer **142** to generate a nonlinear acoustic wave with a finite amplitude creating acoustic streaming. For example, frequencies as low as about 20 kHz may be used. In this connection, it should be noted that acoustic streaming is generated by a nonlinear acoustic wave with a finite amplitude propagating in a viscid fluid. The fluid volume elements of molecules are forced to oscillate at the same frequency as the incident acoustic wave. Due to the nature of the nonlinearity of the acoustic wave, the second-order effect of the wave propagation produces a time-independent flow velocity (DC flow) in addition to a regular oscillatory motion (AC motion). Consequently, the fluid moves in a certain direction, which depends on the geometry of the system and its boundary conditions, as well as the parameters of the incident acoustic wave.

In some embodiments, ocular acoustic device **120** may comprise an imaging module **126** being configured and operable to collect image data being indicative of the displacement of the particles. Controller **124** may be connected to imaging module **126** for receiving the image data. Imaging module **126** is configured to enable visualization of the anterior chamber during the displacement of the particles.

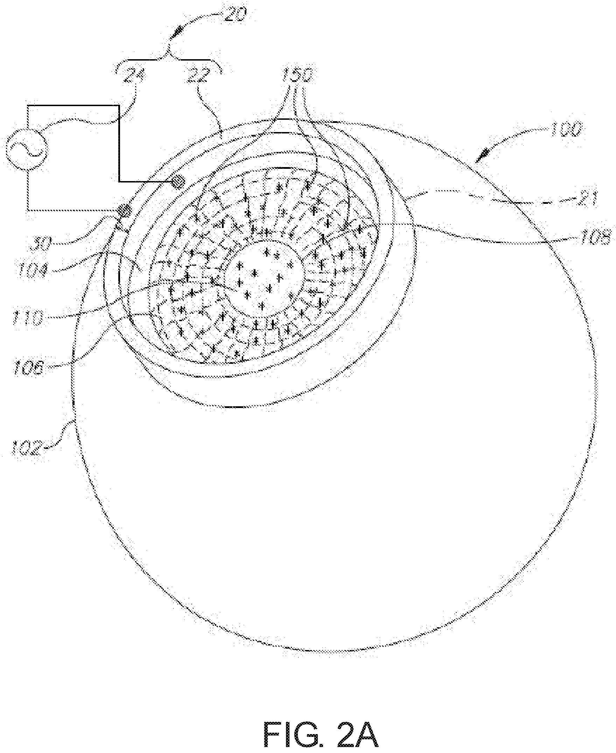

**Fig. 2A** schematically shows an ocular acoustic device **20** positioned on eye **100** for use in controlling movement and position of particles (e.g. **150** shown in **Fig. 1D**), in accordance with an embodiment of the disclosure. Ocular acoustic device **20** comprises an acoustic transducer **22** being configured and operable to generate acoustic waves upon excitation and a controller **24** being configured and operable to excite vibrations in the acoustic transducer **22** generating acoustic waves being capable to enter into the eye **100** and produce an acoustic field in the eye **100** having a directional acoustic energy in a certain pattern that operates to apply forces to the particles. In a specific and non-limiting example, transducer **22** is be formed from PZT (lead zirconate titanate) and has cylindrical, shell an inner diameter equal to about 22 mm, an outer diameter equal to about 26 mm and height equal to about 20 mm.

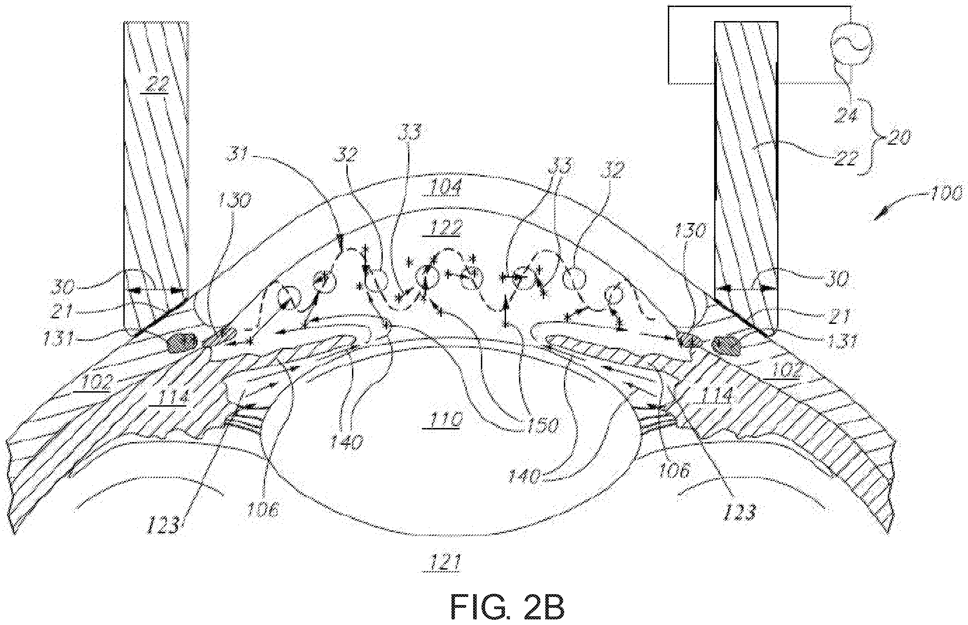

In an embodiment, controller **24** applies voltage at certain frequencies to transducer **22** to excite transverse vibration modes represented by double headed arrows **30** in transducer **22** that generate standing waves in anterior chamber **122 (****Fig. 2B****)** having nodal regions to which particles **150** drift and agglomerate in accordance with an embodiment of the disclosure.

Optionally, controller **24** excites transducer **22** at excitation frequencies between about 250 kHz and about 50 MHz to generate transverse vibration modes **30** in transducer **22** that transmit ultrasound radiation into anterior chamber **122** at wavelengths between about 6 mm and about 30 µm, respectively. The transmitted ultrasound generates standing waves in the anterior chamber having nodal regions separated by distances between about 3 mm and 15 µm. Optionally controller **24** excites transducer **22** at excitation frequencies between about 500 kHz and about 10 MHZ. In an embodiment controller **24** excites transducer **22** at excitation frequencies between about 1MHz and about 5 MHZ.

In an embodiment controller **24** may excite transducer **22** at frequencies as low as about 20 kHz to generate streaming in the anterior chamber.

In some embodiments, ocular acoustic device **20** comprises a coupling layer **21** being configured and operable to physically couple between acoustic transducer **22** and the eye **100** and provide acoustic impedance match thereof. However, the device of the present invention is not limited to this configuration and the acoustic signals may be transmitted directly via an air media (i.e. free-space propagation). Coupling layer **21** thus constitutes the media surrounding the eye **100** and the transducer **22.** The media is not limited and may include at least one of water, air, gel, water solution (BSS,NaCl) ... Transducer **22** is configured to be placed on the eye **100** so that coupling layer **21** is formed on an edge surface of the transducer seats on sclera **102** on or near and substantially concentric with the limbus (not shown) of the cornea. Coupling layer **21** may be formed from a relatively soft resilient material, such as a suitable silicone rubber, that operates to provide acoustic impedance matching of transducer **22** to the eye. The shape of the coupling layer **21** is configured to optimally couple between the transducer **22** and the eye. To this end, it may have a cut closed-loop configuration, when the cut portion corresponds the high portion of the eye.

In an embodiment coupling layer **21** is configured to substantially seal the transducer **22** to eye **100** so that the transducer **22** may be filled with and hold a liquid as an acoustic coupling agent, such as a saline solution or water, on the eye that facilitates generation and transmission of an acoustic field in the eye. The transducer **22** may be implemented as a portable resonator integrated into a water container.

As noted above, coupling layer **21** may be formed from and/or covered by a material suitable for contact with eye **100** and providing acoustic coupling of waves generated by transducer **22** to the eye. For example, coupling layer **21** may be covered by an ultrasound coupling gel to facilitate coupling of ultrasound vibrations excited in transducer **22** to sclera **102** of eye **100.**

**Fig. 2B** schematically shows a cross section view of a portion of eye **100** comprising anterior chamber **122** and lens **110** with acoustic device **20** mounted to the eye and being operated to generate a standing wave pattern **31** characterized by substantially nodal and antinodal regions in anterior chamber **122.** The figure schematically shows a cross-section of the standing wave pattern **31** that excitation of transducer **22** generates in anterior chamber **122** of eye **100.** Circles **32** in the figure indicate nodal regions of standing wave pattern **31,** and arrows **33** represent forces that acoustic pressure generated by the standing wave pattern applies to particles **150,** in accordance with an embodiment of the disclosure. Forces **33** point towards nodal regions **32** and generate drift velocities that move particles **150** toward the nodal regions where the particles tend to agglomerate.

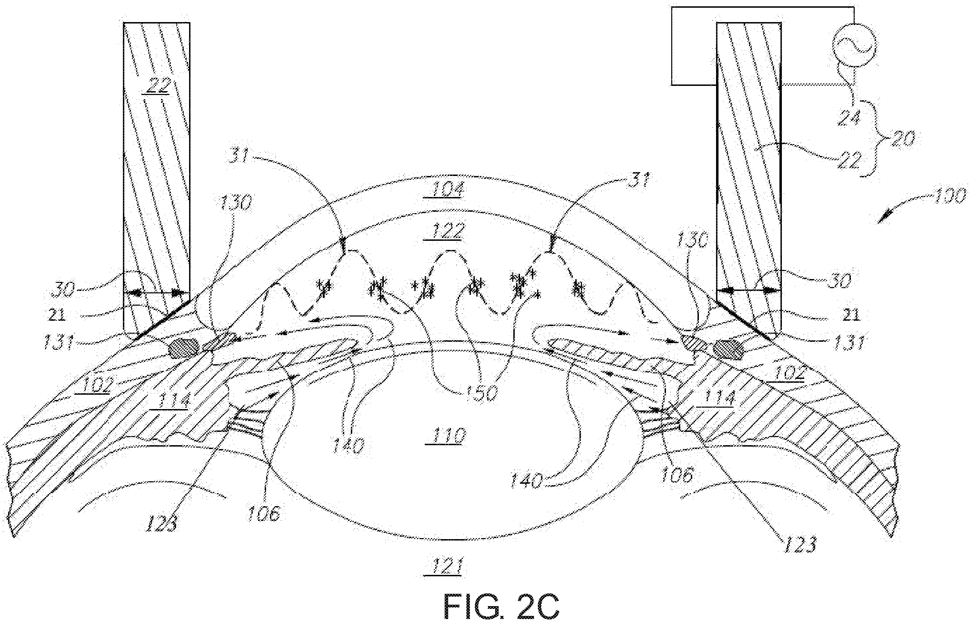

**Fig. 2C** schematically shows the cross-section view shown in **Fig. 2B** after agglomeration of particles **150** at nodal regions **32** of standing wave pattern **31.**

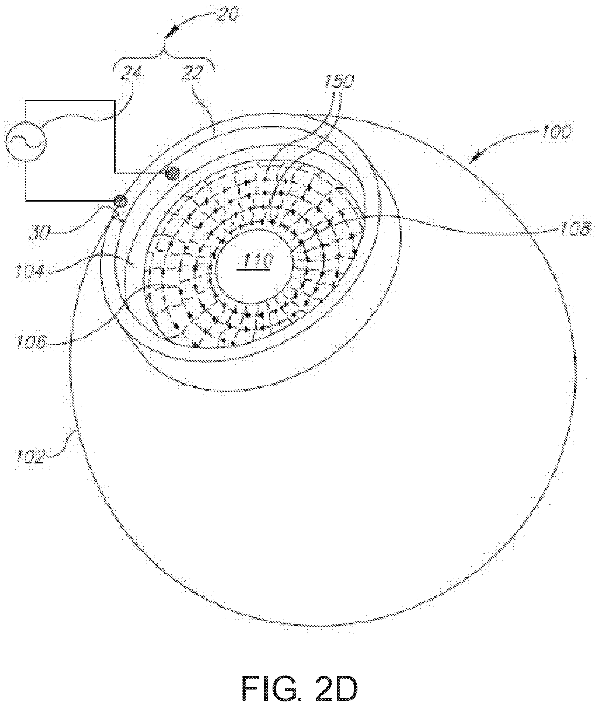

**Fig. 2D** schematically shows a perspective view of acoustic device **20** and eye **100** after agglomeration of particles **150,** in the concentric substantially circular nodal regions of standing wave pattern **31** shown in **Figs. 2B** and **2C****.** The circular nodal regions indicated by circles **32** in the cross-section view shown in **Fig. 2B** are represented by corresponding rings, also labeled by the numeral **32,** in the perspective view of **Fig. 2D****.**

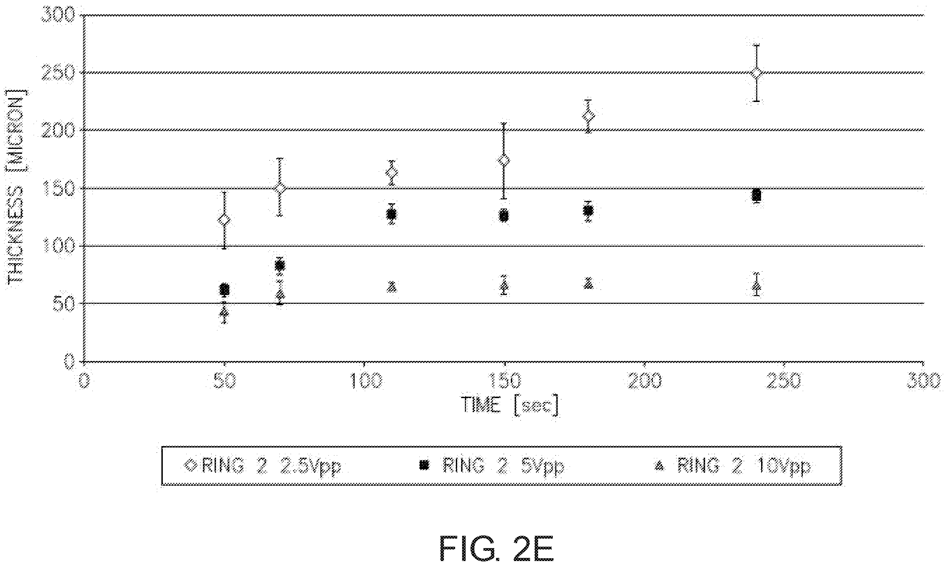

**Fig. 2E** shows a graph of growth of thickness as a function of time of a ring **106 (****Fig. 2D****)** of aggregated particles in a configuration of concentric rings **32** of aggregated particles similar the configuration schematically shown in **Fig. 2D****,** for an experiment carried out on an enucleated porcine eye (not shown) using the teachings of the acoustic device of the present invention. In this specific and non-limiting example, the controller was implemented by using a function generator (Siglent, SDG2082x) generating a sine wave at a frequency of 989 KHz (at resonance of resonator). The acoustic transducer may be implemented by using a hemispherical acoustic resonator (Siasonic, QN19) having 19.5mm diameter, and about 20mm focal length. The starting position of the acoustic transducer was defined as (X=+9mm, Y=+6mm, Z=+6mm, rotation=10°±5°, tilt=0°), the scanning distance was about 12mm (Y or Z axis), and the scanning speed was selected to be about 12mm/min. In the experiment particles of homogenized porcine iris pigment homogenized with or triamcinolone acetonide were injected into anterior chamber of the porcine eye via a clear corneal incision using a 30G needle. Acoustic device **20** was placed in contact with the eye and the lumen of the generator filled with a saline solution. A power supply was operated to excite vibration in the acoustic transducer at a frequency of about 1.1±0.1 MHz and peak to peak AC excitation voltages of 2.5Vpp, 5Vpp, and 10Vpp. Graph of **Fig.2E** shows growth in thickness of a concentric ring of aggregated iris particles second from a center of concentric rings of aggregated particles as a function of time for each of the excitation voltages. The aggregation ring showed growth in thickness over time for excitation at all voltages. For excitation at 10Vpp the ring exhibit relatively strong linear positive correlation to aggregation from a time of 50s (seconds) to a time of 250s.

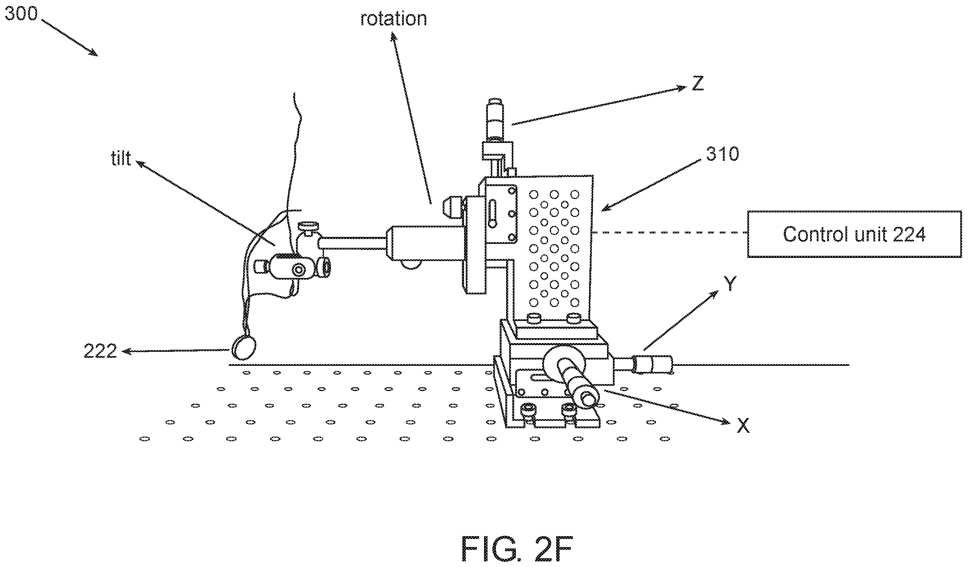

**Fig. 2F** shows a configuration of acoustic device **300** comprising a motion stage **310** holding the acoustic transducer **222.** Motion stage **310** is configured and operable to controllably position and manipulate the acoustic transducer **222** on the eye along five degrees of freedom. Although motion stage **310** has a mechanical configuration, the control of the position of the acoustic transducer **222** may be performed by any electronic and/or mechanical module. Although not shown, motion stage **310** may comprise an electrical motor being able to electrically operate at least one axis of the motion stage. The axes X, Y, Z and the axes of tilt and rotation are shown in the figure. This configuration enables controlled movement of the acoustic transducer by five different axes with constant direct visualization of the anterior chamber. For example, for a transducer having an external perimeter shape of about 10 mm, the motion of the transducer is in the range of 20 mm. The manipulation of the acoustic device **300** may be performed manually or automatically along the eye. Motion stage **31** may be connected (by wires or wireless) to controller **224** being configured and operable to automatically displace acoustic transducer **222** along a desired path. Controller **224** may synchronize between the three-dimensional displacement of acoustic transducer **222** and the different parameters of the acoustic field (e.g. amplitudes and/or phases and/or frequency) to generate and displace the directional pattern. The displacement of the particles from a region to another may be performed by concurrently activating the acoustic field and scanning the surface of the eye along the path defined between the two regions at a certain scanning speed. A plurality of scans may be repetitively performed on the same surface to completely clear the region from particles.

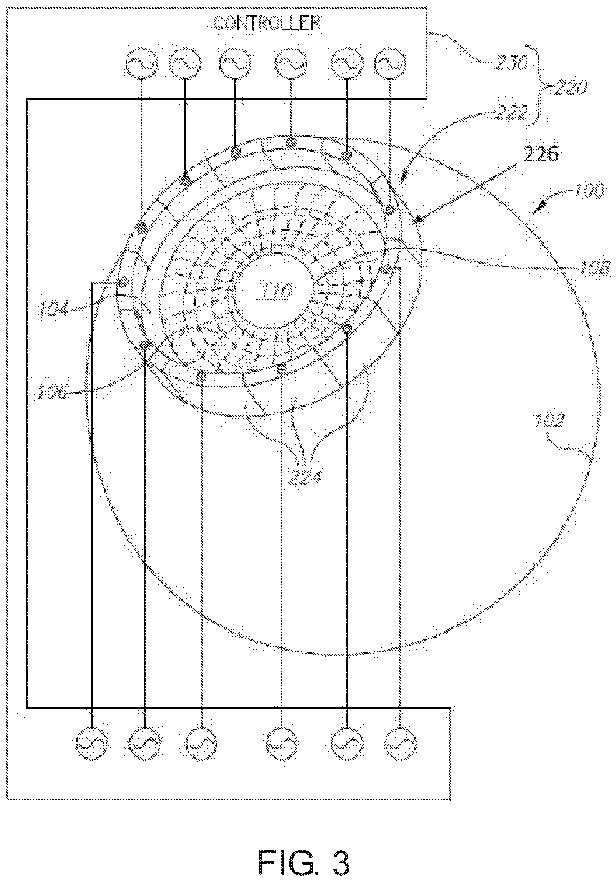

**Fig. 3** schematically shows an acoustic device **220** comprising an optionally cylindrical shell shaped phased array acoustic transducer **222** and a controller **230** operable to excite vibrations in transducer **222** to generate different radiation configurations in eye **100,** in accordance with an embodiment of the disclosure. Transducer **222** comprises a plurality of acoustic transducers **224** each of which is independently excitable by controller **230** to vibrate at selectable amplitudes, frequencies, and phases. A coupling layer **226** of transducer array **222** may be shaped to fit to the surface of eye **100** so that each of acoustic transducers **224** is acoustically coupled to the eye.

In the above description, acoustic devices **20** and **220** are shaped as cylindrical shells and are excited to generate acoustic standing waves that trap and agglomerate particles present in the eye at nodes of the standing waves. However, practice of an embodiment of the disclosure is not limited to cylindrical shell acoustic transducers or to trapping particles at nodes of an acoustic standing wave.

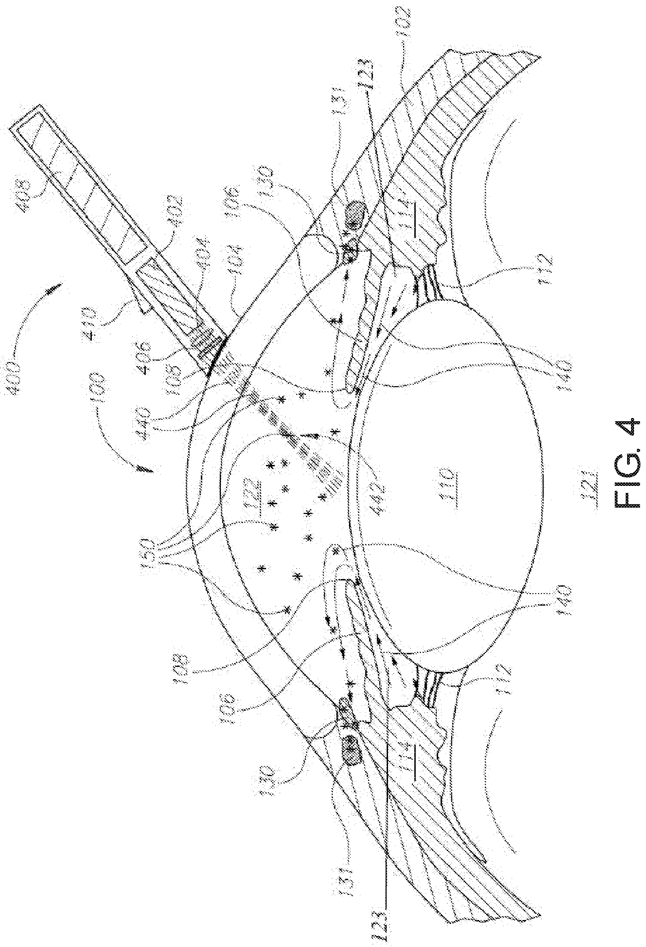

**Fig. 4** schematically shows an acoustic device **400** optionally comprising an acoustic transducer **402** and collimating lens **404** that generate a planar acoustic wave, an acoustic hologram **406** on which the plane wave is incident, and a controller **408** for powering the transducer, in accordance with an embodiment of the disclosure. Although collimating lens **404** and an acoustic hologram **406** are shown in the same figure, the present invention is not limited to this configuration. Collimating lens **404** and an acoustic hologram **406** may be used together or independently in the device of the present invention. Acoustic hologram **406** is optionally configured to convert acoustic energy in the incident planar wave to an acoustic traveling wave **440** that is focused to a narrow focal waist **442** of relatively intense acoustic energy. Focal waist **442** when located in fluid in the anterior, posterior, or vitreous chamber of the eye may be used as an acoustic tweezer to capture and control motion and position of a particle in the chamber. Optionally, acoustic device **400** is configured to be manually operated and comprises an activation button **410** that may be manually operated to turn on and turn off the acoustic device.

In **Fig. 4** acoustic device **400** is schematically shown, optionally manually, placed on cornea **104** of eye **100** to couple and introduce traveling wave **440** into anterior chamber **122** of eye **100** and trap a particle **450** in the focal region. Particle **450** may be moved to a desired location in the eye in accordance with an embodiment by moving stylus acoustic device along cornea **104.**

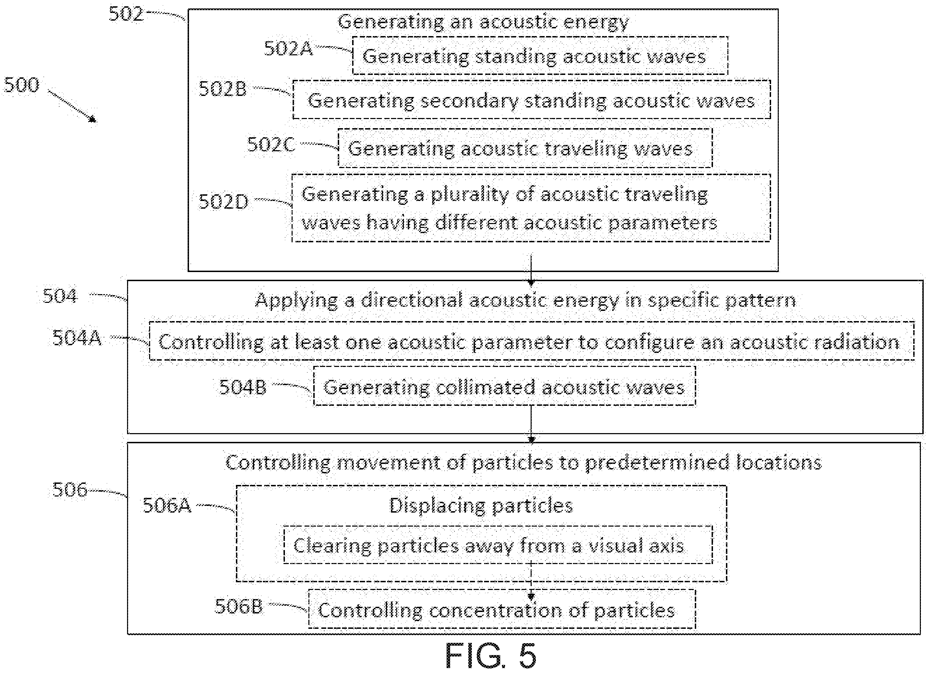

**Fig. 5** is a flow chart showing the main steps of the method for controlling movement and position of particles present in an eye according to some embodiments of the present invention. Method **500** comprises generating acoustic waves in **502** being capable to enter into the eye, producing an acoustic field in the eye having a directional acoustic energy in a certain pattern in **504** that operates to apply forces to the particles and controlling movement of particles in **506** to at least one predetermined location. The directional acoustic field is created in a certain region in which the particles in the eye are attracted and trapped. Generating acoustic waves in **502** may comprise at least one of generating an acoustic standing wave in the eye in **502A;** generating corneal standing acoustic waves in **502B** by generating an acoustic wave being configured to excite the cornea to create vibrations that in turn induces a corneal standing acoustic wave within the anterior chamber of the eye; generating an acoustic travelling wave in the eye in **502C** or generating a plurality of acoustic waves having different acoustic parameters in **502D.** The appropriate selection of the angle at which the acoustic wave is projected onto the eye and of the three-dimensional position of the acoustic transducer relatively to the eye (e.g. distance between the acoustic transducer and the eye) enables to fulfill the conditions for reflection of the acoustic wave and creation of the corneal standing acoustic wave. For example, in **502C,** the rotation angle may be about 15°, the tilt angle 0°, and the three-dimensional position of the acoustic transducer relatively to the eye may be about: X=+9mm, Y=+6mm, Z=+6mm. The reference point (where all the axes are null) is defined where the bottom of the resonator is in contact with connection of the cornea to the sclera at the most righthand side. For example, in **502D** excitation voltages may be increased to increase a thickness of agglomerated particles as a function of time. Producing an acoustic field in the eye having a directional acoustic energy in a certain pattern in **504** enables to apply a directional acoustic energy in specific pattern to the external surface of an eye to create an acoustic pressure within the eye. This may be implemented by controlling at least one acoustic parameter of the acoustic waves in **504A** to configure the acoustic radiation. Alternatively or additionally, applying a directional acoustic energy in specific pattern may comprise generating a collimated beam of acoustic wave energy in **504B** to focus the acoustic energy to a desired internal location in the eye. The acoustic wave energy may be travelling or standing wave. Controlling movement of particles in **506** to at least one predetermined location may comprise displacing particulate matter in the eye in **506A** to be able for example clearing the particles away from the visual axis and reducing their accumulation within the trabecular meshwork by concentrating them in **506B** in a designated area.

Descriptions of embodiments of the invention in the present description are provided by way of example and are not intended to limit the scope of the invention. The described embodiments comprise different features, not all of which are required in all embodiments. Some embodiments utilize only some of the features or possible combinations of the features. Variations of embodiments of the invention that are described, and embodiments comprising different combinations of features noted in the described embodiments, will occur to persons of the art. The scope of the invention is limited only by the claims.

## Claims

1. An ocular acoustic device (20, 120) for controlling movement and position of particles present in an eye, the acoustic device comprising:
an acoustic transducer (22, 142) being configured and operable to generate acoustic waves upon excitation; and
a controller (124) being configured and operable to excite vibrations in the acoustic transducer (22, 142) generating acoustic waves being capable to enter into the eye and produce an acoustic field in the eye having a directional acoustic energy in a certain pattern that operates to apply forces to the particles;
wherein the controller (24, 124) is configured and operable for generating and configuring acoustic radiation for a certain period of time;
**characterized in that** the controller (24, 124) is capable of controlling movement and concentration of particles by controlling at least one acoustic parameter and the certain period of time, wherein the controlling of the movement and concentration of particles comprises causing the particles to drift to and/or cluster at particular regions of the eye, or to attract or repel one another.

2. The ocular acoustic device (20, 120) according to claim 1, wherein the controller (24, 124) is configured and operable to at least one of (i) excite vibrations in the acoustic transducer at a frequency that generates an acoustic standing wave pattern; (ii) excite vibrations in the acoustic transducer (22, 142) generating at least one of acoustic waves exciting the cornea to create vibrations that in turn induces a standing corneal acoustic wave within the anterior chamber of the eye or a nonlinear focused traveling acoustic wave with a finite amplitude creating an acoustic streaming dislocating the particles; or (iii) determine a starting position of the acoustic transducer (22, 142), a scanning path, a scanning speed and at least one acoustic parameter of the acoustic field according to an image data.

3. The ocular acoustic device (120) according to claim 2, wherein the controller (124) is operable to generate an acoustic standing wave pattern comprising generating at least one nodal region to which the particles in the eye are attracted and trapped.

4. The ocular acoustic device (120) according to claim 3, wherein the controller (124) is operable to modify the position of the nodes in the acoustic standing wave pattern.

5. The ocular acoustic device (120) according to any one of the preceding claims, wherein the acoustic transducer (142) comprises at least one of (i) at least one acoustic resonator (142A) or (ii) a plurality of acoustic transducers (142B).

6. The ocular acoustic device (120) according to any one of the preceding claims wherein said acoustic transducer (142) is configured as a probe-like device being configured and operable to control over the direction of particle movement by displacement.

7. The ocular acoustic device (120) according to claim 5, wherein said controller (124) is configured and operable to at least one of (i) excite vibrations in each transducer of the plurality of acoustic transducers (142B) independently; or (ii) control at least one acoustic parameter comprising amplitudes, phases or frequencies of each transducer so that the acoustic transducers (142B) operate as a phased array to generate the acoustic field in the eye.

8. The ocular acoustic device (120) according to any one of the preceding claims, further comprising at least one of (i) a coupling layer (21) being configured and operable to physically couple between the acoustic transducer (142) and the eye and provide acoustic impedance match thereof; (ii) an acoustic hologram (128) being configured to receive acoustic waves generated by the acoustic transducer (142) and configure the received wave to form an acoustic field in the eye; (iii) a collimating lens (404) being capable of receiving the acoustic waves generated by the acoustic transducer (142) and generating a collimated beam of traveling wave acoustic energy; (iv) a motion stage (310) being capable of holding the acoustic transducer (142); the motion stage (310) being configured and operable to controllably position and manipulate the acoustic transducer (142) on the eye; (v) a housing that houses at least one or any combination of more than one of the acoustic transducers, acoustic lens, and/or hologram; or (vi) an imaging module (126) being configured and operable to collect image data being indicative of the displacement of the particles, wherein said controller (124) is connected to said imaging module (126) for receiving the image data.

## Patentansprüche

1. Eine akustische Augenvorrichtung (20, 120) zur Steuerung der Bewegung und Position von Partikeln in einem Auge, wobei die akustische Vorrichtung Folgendes umfasst:
einen akustischen Wandler (22, 142), der konfiguriert ist und arbeitet, um bei Erregung akustische Wellen zu erzeugen; und
eine Steuerung (124), die konfiguriert ist und arbeitet, um Schwingungen im akustischen Wandler (22, 142) zu erzeugen und die akustische Wellen erzeugt, die in der Lage sind, in das Auge einzudringen und ein akustisches Feld im Auge zu erzeugen, das gerichtete akustische Energie in einem bestimmten Muster hat, welches wirkt, um Kräfte auf die Partikel auszuüben;
wobei die Steuerung (24, 124) konfiguriert ist und arbeitet, um für einen bestimmten Zeitraum Schallstrahlung zu erzeugen und zu konfigurieren;
**dadurch gekennzeichnet, dass** die Steuerung (24, 124) in der Lage ist, die Bewegung und Konzentration von Partikeln zu steuern durch Steuerung mindestens eines akustischen Parameters und des bestimmten Zeitraums, wobei das Steuern der Bewegung und Konzentration von Partikeln das Veranlassen der Partikel umfasst, zu wandern und/oder sich in bestimmten Bereichen des Auges anzusammeln oder einander anzuziehen oder abzustoßen.

2. Die akustische Augenvorrichtung (20, 120) gemäß Anspruch 1, wobei die Steuerung (24, 124) konfiguriert ist und arbeitet, um mindestens eines von Folgendem durchzuführen: (i) Schwingungen im akustischen Wandler mit einer Frequenz zu erzeugen, die ein akustisches stehendes Wellenmuster erzeugt; (ii) Schwingungen im akustischen Wandler (22, 142) zu erzeugen, die mindestens eines von Folgendem generieren: akustische Wellen, die aus der Hornhaut austreten, um Schwingungen zu erzeugen, die wiederum eine stehende akustische Hornhaut-Welle in der vorderen Augenkammer erzeugt, oder eine nicht lineare fokussierte akustische Wanderwelle mit begrenzter Amplitude, die ein akustisches Strömen auslöst, welches die Partikel verschiebt; oder (iii) eine Startposition des akustischen Wandlers (22, 142), einen Scanning-Pfad, eine Scanning-Geschwindigkeit und mindestens einen akustischen Parameter des akustischen Feldes entsprechend einem Bilddatum zu bestimmen.

3. Die akustische Augenvorrichtung (120) gemäß Anspruch 2, wobei die Steuerung (124) arbeitet, um ein akustisches stehendes Wellenmuster zu erzeugen, das das Erzeugen mindestens eines Knotenbereichs umfasst, von welchem die Partikel im Auge angezogen und eingefangen werden.

4. Die akustische Augenvorrichtung (120) gemäß Anspruch 3, wobei die Steuerung (124) arbeitet, um die Position der Knoten im akustischen stehenden Wellenmuster zu verändern.

5. Die akustische Augenvorrichtung (120) gemäß einem beliebigen der obigen Ansprüche, wobei der akustische Wandler (142) mindestens eines von Folgendem umfasst: (i) mindestens einen Acoustic Resonator (142A) oder (ii) eine Vielzahl akustischer Wandler (142B).

6. Die akustische Augenvorrichtung (120) gemäß einem beliebigen der obigen Ansprüche, wobei der akustische Wandler (142) als sondenartige Vorrichtung konfiguriert ist, die konfiguriert ist und arbeitet, um die Richtung der Partikelbewegung durch Verschiebung zu steuern.

7. Die akustische Augenvorrichtung (120) gemäß Anspruch 5, wobei die Steuerung (124) konfiguriert ist und arbeitet, um (i) Schwingungen in jedem Wandler der Vielzahl akustischer Wandler (142B) unabhängig zu erzeugen; und/oder (ii) mindestens einen akustischen Parameter zu steuern, der Amplituden, Phasen oder Frequenzen jedes Wandlers umfasst, so dass die akustischen Wandler (142B) als Phased Array arbeiten, um das akustische Feld im Auge zu erzeugen.

8. Die akustische Augenvorrichtung (120) gemäß einem beliebigen der obigen Ansprüche, die weiter mindestens eines von Folgendem umfasst: (i) eine Kopplungsschicht (21), die konfiguriert ist und arbeitet, um eine physische Kopplung zwischen dem akustischen Wandler (142) und dem Auge herzustellen und eine akustische Impedanzanpassung derselben durchzuführen; (ii) ein akustisches Hologramm (128), das konfiguriert ist, um akustische Wellen zu empfangen, die vom akustischen Wandler (142) erzeugt werden, und die empfangene Welle zu konfigurieren, um ein akustisches Feld im Auge zu bilden; (iii) eine Kollimationslinse (404), die in der Lage ist, die akustischen Wellen zu empfangen, die vom akustischen Wandler (142) erzeugt werden, und einen kollimierten Strahl akustischer Wanderwellenenergie zu erzeugen; (iv) eine Bewegungsstufe (310), die in der Lage ist, den akustischen Wandler (142) zu halten; wobei die Bewegungsstufe (310) konfiguriert ist und arbeitet, um den akustischen Wandler (142) steuerbar auf dem Auge zu positionieren und zu handhaben; (v) ein Gehäuse, das mindestens eines oder eine beliebige Kombination von Folgendem enthält: der akustischen Wandler, der akustischen Linse und/oder des Hologramms; oder (vi) ein Abbildungsmodul (126), das konfiguriert ist und arbeitet, um Bilddaten zu sammeln, die die Verschiebung der Partikel anzeigen, wobei die Steuerung (124) mit dem Abbildungsmodul (126) zum Empfangen der Bilddaten verbunden ist.

## Revendications

1. Dispositif acoustique oculaire (20, 120) destiné à contrôler le mouvement et la position de particules présentes dans un œil, le dispositif acoustique comprenant :
un transducteur acoustique (22, 142) configuré et apte à générer des ondes acoustiques lorsqu'il est excité ; et
un contrôleur (124) configuré et apte à exciter des vibrations dans le transducteur acoustique (22, 142) générant des ondes acoustiques capables de pénétrer dans l'œil et de produire un champ acoustique dans l'œil présentant une énergie acoustique directionnelle selon un certain schéma qui agit de manière à appliquer des forces aux particules ;
où
le contrôleur (24, 124) est configuré et apte à générer et à configurer rayonnement acoustique pendant une certaine durée;
**caractérisé en ce que** le contrôleur (24, 124) est capable
de contrôler mouvement et concentration des particules en régulant au moins un paramètre acoustique et ladite certaine durée, où le contrôle du mouvement et de la concentration des particules comporte le fait d'amener les particules à dériver vers et/ou à s'agglomérer dans des régions particulières de l'œil, ou à s'attirer ou à se repousser les unes les autres.

2. Dispositif acoustique oculaire (20, 120) selon la revendication 1, où le contrôleur (24, 124) est configuré et apte à effectuer au moins l'une des opérations suivantes : (i) exciter des vibrations dans le transducteur acoustique à une fréquence qui génère un motif d'onde stationnaire acoustique ; (ii) exciter des vibrations dans le transducteur acoustique (22, 142) générant au moins l'une des ondes acoustiques excitant la cornée pour créer des vibrations qui, à leur tour, induisent une onde acoustique cornéenne stationnaire au sein de la chambre antérieure de l'œil ou une onde acoustique progressive focalisée non linéaire d'amplitude finie créant un courant acoustique déplaçant les particules ; ou (iii) déterminer une position de départ du transducteur acoustique (22, 142), un trajet de balayage, une vitesse de balayage et au moins un paramètre acoustique du champ acoustique en fonction de données d'image.

3. Dispositif acoustique oculaire (120) selon la revendication 2, où le contrôleur (124) est apte à générer un motif d'onde acoustique stationnaire comprenant la génération d'au moins une région nodale vers laquelle les particules présentes dans l'œil sont attirées et piégées.

4. Dispositif acoustique oculaire (120) selon la revendication 3, où le contrôleur (124) est apte à modifier la position des nœuds dans le motif d'onde stationnaire acoustique.

5. Dispositif acoustique oculaire (120) selon l'une quelconque des revendications précédentes, où le transducteur acoustique (142) comprend au moins l'un des éléments suivants : (i) au moins un résonateur acoustique (142A) ou (ii) une pluralité de transducteurs acoustiques (142B).

6. Dispositif acoustique oculaire (120) selon l'une quelconque des revendications précédentes, où ledit transducteur acoustique (142) est configuré comme un dispositif de type sonde, configuré et apte à contrôler la direction du mouvement des particules par déplacement.

7. Dispositif acoustique oculaire (120) selon la revendication 5, où ledit contrôleur (124) est configuré et apte à effectuer au moins l'une des opérations suivantes : (i) exciter indépendamment des vibrations dans chaque transducteur de la pluralité de transducteurs acoustiques (142B) ; ou (ii) de contrôler au moins un paramètre acoustique comprenant les amplitudes, les phases ou les fréquences de chaque transducteur, de sorte que les transducteurs acoustiques (142B) fonctionnent comme un réseau phasé pour générer le champ acoustique dans l'œil.

8. Dispositif acoustique oculaire (120) selon l'une quelconque des revendications précédentes, comprenant en outre au moins l'un des éléments suivants : (i) une couche de couplage (21) configurée et apte à assurer le couplage physique entre le transducteur acoustique (142) et l'œil et à assurer l'adaptation d'impédance acoustique entre ceux-ci ; (ii) un hologramme acoustique (128) configuré pour recevoir les ondes acoustiques générées par le transducteur acoustique (142) et configurer l'onde reçue afin de former un champ acoustique dans l'œil ; (iii) une lentille de collimation (404) capable de recevoir les ondes acoustiques générées par le transducteur acoustique (142) et de générer un faisceau collimaté d'énergie acoustique à onde progressive ; (iv) une plateforme de mouvement (310) capable de supporter le transducteur acoustique (142) ; la plateforme de mouvement (310) étant configuré et pouvant fonctionner pour positionner et manipuler de manière contrôlée le transducteur acoustique (142) sur l'œil ; (v) un boîtier logeant au moins un ou toute combinaison de plusieurs des éléments suivants : transducteurs acoustiques, lentille acoustique et/ou hologramme ; ou (vi) un module d'imagerie (126) configuré et apte à collecter des données d'image indiquant le déplacement des particules, où ledit contrôleur (124) est connecté audit module d'imagerie (126) pour recevoir les données d'image.
